# FASCICULE DE BREVET EUROPEEN

(11) **EP 2 365 760 B1**
(45) Date de publication et mention de la délivrance du brevet: **20.11.2013**
(21) Numéro de dépôt: 09783849.4
(22) Date de dépôt: 08.10.2009
(51) Int. Cl.: A23L 1/221, A61K 8/33, A61K 31/11, C07C 47/58, A61K 8/34, A61Q 13/00, C07C 45/81, C07C 47/575

(54) **NOUVEAU COMPOSE A BASE DE VANILLINE ET D'ETHYLVANILLINE, SA PREPARATION ET SES APPLICATIONS**
NEUE VERBINDUNG, DIE VANILLIN UND ETHYLVANILLIN ENTHÄLT, UND IHRE HERSTELLUNG UND ANWENDUNGEN
NOVEL COMPOUND CONTAINING VANILLIN AND ETHYLVANILLIN, AND PREPARATION AND APPLICATIONS THEREOF

(30) Priorité: 24.10.2008 FR 0805913
(43) Date de publication de la demande: 21.09.2011
(73) Titulaire: Rhodia Opérations, 93306 Aubervilliers (FR)
(72) Inventeur: LE-THIESSE, Jean-Claude, F-42100 Saint-Etienne (FR)
(74) Mandataire: Blanchard, Isabelle Jackie
(86) Numéro de dépôt international: PCT/EP2009/063093
(87) Numéro de publication internationale: WO 2010/046239

(56) Documents cités:
- DATABASE CA CHEMICAL ABSTRACTS SERVICE, COLUMBUS, OHIO, US; 1965, ZENON, RYSZARD ET AL: "Food essence with vanilla flavor" XP002533462 extrait de STN Database accession no. 1969:402311 cité dans la demande
- [en ligne] Extrait de l'Internet: <URL:http://fr.wikipedia.org/wiki/Pascal_(u nité)>

## Description

La présente invention a pour objet un nouveau composé à base de vanilline et d'éthylvanilline et son procédé de préparation.

Plus précisément, l'invention a trait à un nouveau composé obtenu par co-cristallisation de vanilline et d'éthylvanilline.

L'invention vise également sa mise en oeuvre dans de nombreux domaines d'application, notamment dans l'alimentation humaine et animale.

La vanilline ou 4-hydroxy-3-méthoxybenzaldéhyde est un produit largement utilisée dans de nombreux domaines d'application en tant qu'arôme et/ou parfum.

Ainsi, la vanilline se trouve abondamment consommée dans l'industrie alimentaire et animale mais elle a aussi des applications dans d'autres domaines tels que par exemple, la pharmacie ou la parfumerie. Il s'ensuit que c'est un produit de grande consommation.

La vanilline est très souvent associée à l'éthylvanilline ou 3-éthoxy-4-hydroxybenzaldéhyde car il est connu que la présence d'une faible quantité d'éthylvanilline permet d'exalter les propriétés parfumantes et/ou organoleptiques de la vanilline.

Ainsi, un utilisateur potentiel souhaiterait avoir à disposition un mélange de vanilline et d'éthylvanilline déjà effectué.

Le problème qui se pose est que la préparation dudit mélange réalisée selon une technique classique de mélange à sec de poudres de vanilline et d'éthylvanilline conduit à l'obtention d'un mélange qui présente la propriété de motter d'une façon très importante. Il en résulte l'impossibilité d'utiliser un tel mélange en raison de sa présentation qui n'est pas sous forme pulvérulente et de très grandes difficultés à solubiliser la masse obtenue.

Par ailleurs, un stockage prolongé conduit à une aggravation du phénomène de mottage, aboutissant à une prise en masse de la poudre.

Ainsi, le but de l'invention est de fournir une nouvelle présentation à base de vanilline et d'éthylvanilline présentant des propriétés de coulabilité améliorées et l'absence de mottage au stockage.

Il a maintenant été trouvé et c'est ce qui fait l'objet de la présente invention, un nouveau composé obtenu par co-cristallisation de vanilline et d'éthylvanilline mis en oeuvre dans un ratio molaire vanilline/éthylvanilline de 2.

Un autre objet de l'invention est le procédé d'obtention dudit composé à partir de vanilline et d'éthylvanilline caractérisé par le fait qu'il comprend la co-cristallisation de vanilline et d'éthylvanilline mises en oeuvre dans un ratio molaire vanilline/éthylvanilline de 2, en milieu fondu ou en solution dans un solvant les solubilisant.

Conformément à l'invention, il a été trouvé que le composé obtenu par co-cristallisation de vanilline et d'éthylvanilline dans un ratio molaire de 2 (correspondant à un rapport pondéral 65/35), présentait des caractéristiques qui lui sont propres.

Il se présente sous forme d'une poudre blanche qui a un point de fusion mesuré par analyse calorimétrique différentielle de 60 °C ± 2°C différent de celui de la vanilline et de l'éthylvanilline respectivement de 81 °C ± 1 °C et de 76 °C ± 1 °C.

Le composé de l'invention possède un spectre de diffraction des rayons X qui lui est spécifique et qui est différent de celui de la vanilline et de l'éthylvanilline.

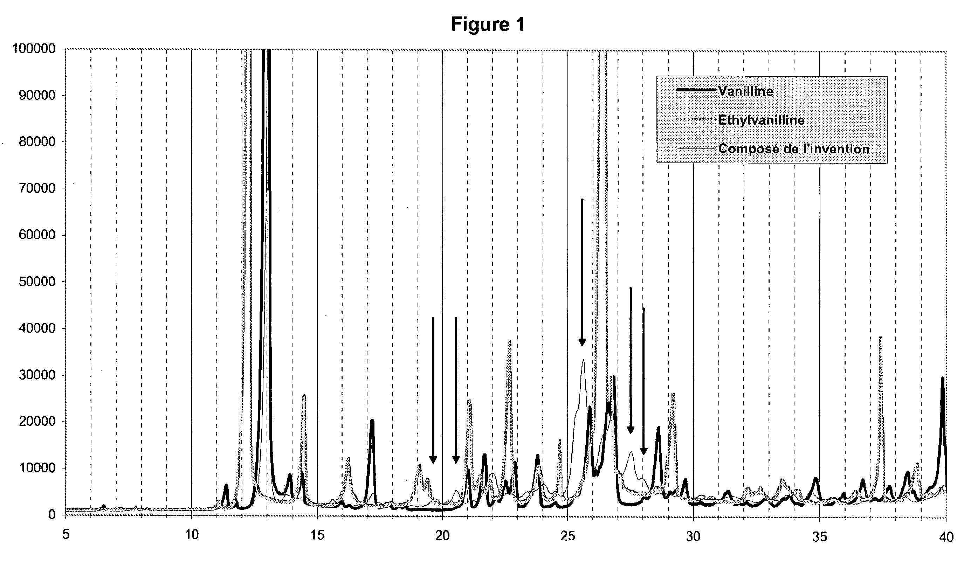

La figure 1 représente trois courbes correspondant aux différents spectres de diffraction des rayons X du composé de l'invention, de la vanilline et de l'éthylvanilline.

Sur le spectre du composé de l'invention à base de vanilline et d'éthylvanilline, on remarque notamment la présence de raies aux angles 2θ (en °) = 20,7 - 25,6 - 27,5 - 28,0 ; lesdites raies étant absentes sur les spectres de diffraction des rayons X de la vanilline et de l'éthylvanilline.

Une autre caractéristique du composé de l'invention est que son spectre de diffraction des rayons X ne subit pas de modification significative au cours d'un stockage prolongé.

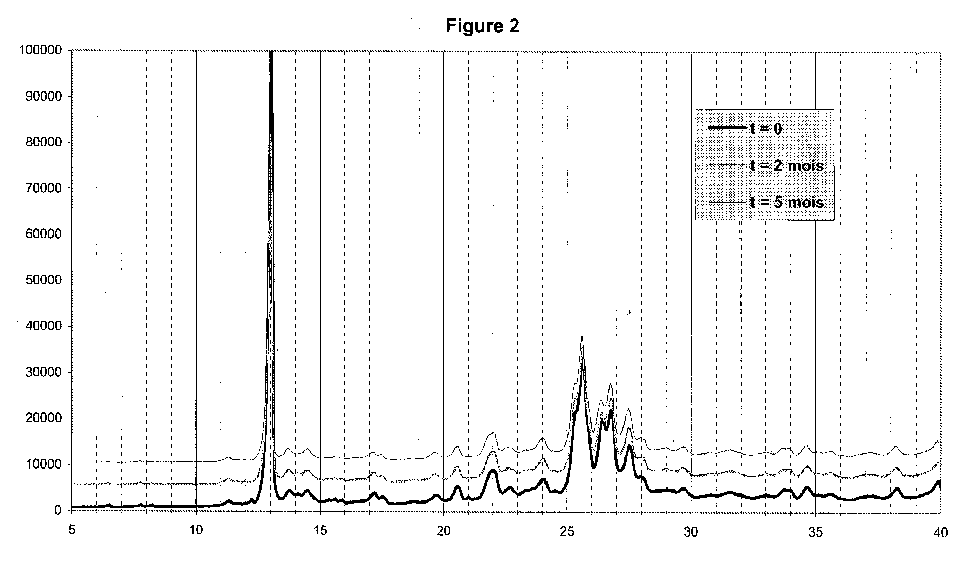

L'évolution de son spectre a été suivie en fonction de la durée de stockage à température ambiante. Sur une période de stockage prolongée (5 mois), on n'observe rigoureusement aucune modification du spectre du composé de l'invention comme le met en évidence la figure 2 qui est explicitée dans l'exemple 1.

On constate une absence de modification des raies spécifiques du composé de l'invention.

Une autre caractéristique du composé de l'invention est que c'est un composé pas ou très peu hygroscopique comme la vanilline et l'éthylvanilline.

L'hygroscopicité du composé de l'invention est déterminée en mesurant sa variation de masse après avoir été maintenu pendant 1 heure, à 40°C sous air à 80 % d'humidité relative.

Ledit composé adsorbe moins de 0,5 % en poids d'eau, sa teneur se situe de préférence entre 0,1 et 0,3 % en poids d'eau. Ledit composé reste parfaitement solide.

Il est à noter que le brevet PL 54 771 décrit un arôme alimentaire comprenant 57 % en poids de vanilline et 43 % en poids d'éthylvanilline. Ce mélange a une composition différente du produit de l'invention et présente des caractéristiques physico-chimiques différentes du produit de l'invention.

Selon PL 54 77, ce mélange dit eutectique possède un point de fusion de 49°C comparé à 60°C pour le produit de l'invention.

Une autre différence majeure est en rapport avec ses propriétés d'hygroscopicité. En effet, un mélange comprenant 57 % en poids de vanilline et 43 % en poids d'éthylvanilline porté à 40°C et sous air à 80 % d'humidité relative, adsorbe plus de 3 % en poids d'eau et devient pâteux voire partiellement liquide dans ces conditions. Il est donc impossible de stocker ou de mettre en oeuvre ce mélange sous des conditions climatiques de température et/ou d'humidité élevées fréquemment rencontrées dans certaines zones géographiques alors que le produit de l'invention reste parfaitement solide et aisément manipulable.

Ainsi, le composé de l'invention présente des propriétés de mottage très améliorées par rapport à un simple mélange à sec de vanilline et d'éthylvanilline.

Ledit mélange à sec avec un rapport massique VA/EVA compris entre 2/98 et 98/2 prend en masse après un stockage à température ambiante (22°C), en moins d'une semaine alors que le composé de l'invention stocké dans les mêmes conditions ne prend pas en masse après un mois, voire même après plusieurs mois (par exemple au moins 6 mois).

Le composé de l'invention présente de bonnes propriétés organoleptiques.

Il possède une puissance aromatique élevée nettement supérieure à celle de la vanilline. Ainsi, dans ses applications en tant qu'arôme, des quantités moindres, par exemple, des quantités divisées par deux peuvent être utilisées sans constater de différence de puissance aromatique.

Les propriétés particulières du composé de l'invention sont liées à deux paramètres à savoir le ratio molaire entre la vanilline et l'éthylvanilline et au fait qu'il y a une co-cristallisation entre la vanilline et l'éthylvanilline sous une forme cristalline spécifique caractérisée par son point de fusion et son spectre de diffraction des rayons X.

Un autre objet de l'invention est donc le procédé d'obtention du composé de l'invention

Conformément à l'invention, il a été trouvé que le nouveau composé de vanilline et d'éthylvanilline présente des propriétés de mottage améliorées dès lors qu'il est obtenu par co-cristallisation de vanilline et d'éthylvanilline mis en oeuvre dans le ratio molaire de 2.

Un premier mode de préparation consiste à effectuer la co-cristallisation de la vanilline et de l'éthylvanilline dans un solvant.

Un autre mode de réalisation consiste à effectuer la co-cristallisation selon une opération de fusion suivie par une solidification par refroidissement à température contrôlée.

Intervient dans le procédé de l'invention, de la vanilline et de l'éthylvanilline dans un ratio molaire vanilline/éthylvanilline de 2 correspondant à un mélange pondéral comprenant 65 % de vanilline et 35 % d'éthylvanilline.

Selon un mode de réalisation de l'invention, on solubilise la vanilline et l'éthylvanilline dans un solvant.

Le solvant susceptible d'être utilisé doit être chimiquement inerte vis-à-vis de la vanilline et de l'éthylvanilline et resté inerte lors du chauffage dans la zone de température définie ci-après.

Comme solvants susceptibles d'être mis en oeuvre dans les compositions de l'invention, on peut faire appel, de préférence, à un solvant polaire, protique ou aprotique ou à un mélange de solvants.

On donne ci-après, des exemples de solvants convenant tout à fait à la présente invention :
- l'eau,
- les alcools, de préférence, aliphatiques ou arylaliphatiques et plus préférentiellement, le méthanol, l'éthanol, le propanol, l'isopropanol, le butanol, l'alcool β-phényléthylique, l'éthylèneglycol, le diéthylèneglycol, le propylèneglycol, le glycérol,
- les éther-oxydes de préférence, aliphatiques, et, plus particulièrement, le diéthyléther, le dipropyléther, le diisopropyléther, le dibutyléther, le méthyltertiobutyléther, l'éthyltertiobutyléther, le ditertiobutyléther, le diméthyléther de l'éthylèneglycol, le diméthyléther du diéthylèneglycol,
- les esters d'alkyle ou d'arylalkyle d'acides carboxyliques, aliphatiques, cycloaliphatiques ou aromatiques, et plus préférentiellement, l'acétate d'éthyle, l'acétate de butyle, le salicylate de benzyle, le laurate de méthyle, le benzoate de méthyle, le citrate d'éthyle, le triacétylglycérol ou triacétine, ester du glycérol et de l'acide acétique.

La liste donnée ci-dessus n'est pas limitative.

Parmi les solvants précités, on choisit préférentiellement l'eau, l'éthanol, le propylèneglycol, la triacétine et leurs mélanges.

En ce qui concerne la quantité de solvant utilisée, sa quantité dépend de la nature du solvant et de la température de solubilisation. Cette quantité est d'autant plus élevée que la température de solubilisation est plus basse.

La quantité de solvant mis en oeuvre, exprimée en poids par rapport au poids de matières sèches (vanilline + éthylvanilline) varie généralement entre 5 et 60 %.

Selon la quantité de solvant mis en oeuvre par rapport au poids de matières sèches, le mélange peut éventuellement être porté à une température de préférence comprise entre 40°C et 90°C et plus préférentiellement entre 50°C et 80°C pour faciliter la dissolution de la vanilline et de l'éthylvanilline.

Selon un autre mode d'exécution, on porte le solvant à la température définie ci-dessus puis l'on introduit la vanilline et l'éthylvanilline dans un ratio molaire de 2.

On maintient le mélange sous agitation jusqu'à obtention d'une solution homogène. Généralement, la durée varie entre 10 et 120 min.

Ensuite, on refroidit la solution ainsi obtenue pour provoquer la cristallisation du composé de l'invention.

Si la solution a été préparée entre 40°C et 90°C, un refroidissement à température ambiante est généralement suffisant mais il peut également être effectué à une température allant jusqu'à 0°C.

Par « température ambiante », on entend une température située entre 15°C et 25°C, de préférence comprise entre 18°C et 22°C.

Si la solution a été préparée à température ambiante, un refroidissement à une température comprise entre 0°C et 10°C et plus préférentiellement entre 0°C et 5°C est nécessaire pour provoquer la cristallisation du composé de l'invention.

Quelle que soit la variante de préparation, on sépare le produit cristallisé selon les techniques classiques de séparation solide/liquide de préférence par filtration ou centrifugation.

On effectue ensuite une opération de séchage qui peut être effectuée dans un dispositif classique de séchage tel que par exemple, un four, un séchoir à plateaux, un lit fluidisé, une étuve sous vide, etc ...

On peut conduire le séchage sous air ou bien sous atmosphère de gaz inertes, de préférence sous atmosphère d'azote. On peut aussi réaliser le séchage dans une enceinte sous pression réduite par exemple sous une pression comprise entre 10 et 500 mm de mercure (soit de 1,33×10³ à 6,66×10⁴ Pa).

Le séchage est réalisé en portant les cristaux du composé obtenu jusqu'à une température de 51°C ± 1 °C.

La durée du séchage varie généralement de 15 min à 2 heures.

On obtient un composé ayant les propriétés définies ci-dessus.

Une variante du procédé de l'invention consiste à préparer le composé de l'invention selon une opération qui consiste à effectuer la fusion du mélange de vanilline et d'éthylvanilline mises en oeuvre dans un ratio molaire de 2 puis le refroidissement du mélange fondu en abaissant la température à 50°C ± 1 °C, puis l'on maintient cette température jusqu'à solidification totale du mélange.

Selon une variante préférée du procédé de l'invention, on effectue le refroidissement, en l'absence de toute agitation.

A cet effet, on charge la vanilline et l'éthylvanilline mis en oeuvre dans un ratio molaire de 2, séparément ou en mélange et l'on porte le mélange à une température qui est choisie entre 60°C et 90°C et qui se situe de préférence entre 70°C et 80°C.

Cette opération est généralement effectuée sous agitation dans un dispositif quelconque, notamment dans une cuve équipée d'un dispositif classique de chauffage comme par exemple un système de chauffage par résistances électriques ou bien par circulation d'un fluide caloporteur dans une double enveloppe ou encore dans une enceinte chauffée telle que four, étuve.

Il est souhaitable d'effectuer la préparation de ce mélange fondu sous atmosphère de gaz inertes qui est préférentiellement de l'azote.

On maintient le mélange à la température choisie jusqu'à obtention du mélange fondu.

Le produit fondu est transféré dans un récipient quelconque, par exemple un plateau en inox, qui permettra de récupérer aisément le produit après solidification. Ce récipient est préchauffé entre 70 et 80°C avant de recevoir le mélange fondu.

Dans une étape suivante, on effectue le refroidissement du mélange fondu jusqu'à une température de 50°C ± 1, par régulation de la température de refroidissement par tout moyen connu.

Comme mentionné précédemment, le refroidissement est effectué de préférence en l'absence de toute agitation.

Le mélange solidifié obtenu peut être mis en forme et différentes techniques peuvent être envisagées.

L'une d'entre elles consiste à effectuer le broyage du mélange obtenu de telle sorte que la taille des particules soit compatible avec l'application envisagée.

Elle s'échelonne le plus souvent entre 100 µm et 2 mm.

Généralement, la taille des particules exprimée par le diamètre médian (d₅₀) varie de 100 µm à 800 µm, de préférence entre 200 µm et 300 µm. On définit le diamètre médian comme étant tel que 50 % en poids des particules ont un diamètre supérieur ou inférieur au diamètre médian.

L'opération de broyage peut être effectuée dans un appareillage classique tel qu'un broyeur à palettes, un broyeur à broches, un granulateur.

Une autre mise en forme peut être effectuée en mettant en oeuvre la technique d'écaillage sur cylindre ou sur bande.

On prépare un mélange fondu de vanilline et d'éthylvanilline dans les proportions précédemment indiquées. Le mélange fondu est ensuite mis en contact avec un cylindre ou une bande métallique refroidie à une température de 50°C, puis en raclant avec un couteau le film obtenu sur le cylindre, on récupère le mélange de vanilline et d'éthylvanilline solide sous forme d'écailles.

Le procédé de l'invention grâce à cette étape de co-cristallisation permet d'obtenir un nouveau composé de vanilline et d'éthylvanilline qui présente des propriétés de stockage améliorées car le phénomène de mottage est fortement réduit comme mis en évidence dans les exemples.

L'invention n'exclut pas la mise en oeuvre d'un ou des excipients avec le composé de l'invention

Il est à noter que le choix du ou des excipients doit tenir compte de la destination du produit final et ainsi présenter la propriété de comestibilité dès lors qu'il est mis en oeuvre dans le domaine alimentaire.

La quantité d'excipient(s) peut être très variable et elle peut représenter de 0,1 à 90 % du poids du mélange final.

Elle est choisie avantageusement entre 20 et 60 % en poids.

Selon le type d'excipient retenu, la quantité utilisée et la destination du produit final, l'excipient peut être soit ajouté par mélange à sec avec le composé de l'invention, soit incorporé dans le procédé d'obtention du composé de l'invention, par exemple lors de l'étape de fusion du mélange vanilline et éthylvanilline.

On donne ci-après des exemples d'excipients susceptibles d'être utilisés qui sont donnés sans caractère limitatif.

Un premier type d'excipients sont les corps gras.

Comme exemples, on peut mentionner les acides gras éventuellement sous forme de sels ou d'esters.

Les acides gras mis en oeuvre sont généralement des acides gras saturés à longue chaîne, c'est-à-dire ayant une longueur de chaîne entre environ 9 et 21 atomes de carbone tels que par exemple, l'acide caprique, l'acide laurique, l'acide tridécylique, l'acide myristique, l'acide palmitique, l'acide stéarique, l'acide béhénique.

Il est possible que lesdits acides soient sous forme salifiée et l'on peut citer notamment le stéarate de calcium ou de magnésium.

Comme esters d'acides gras, on peut citer en particulier le stéarate de glycéryle, le palmitate d'isopropyle, le palmitate de cétyle, le myristate d'isopropyle.

On peut également citer plus spécifiquement, les esters de glycérol et d'acides gras à longue chaîne tels que le monostéarate de glycérol, le monopalmitostéarate de glycérol, le palmitostéarate de glycérol, le palmitostéarate d'éthylèneglycol, le palmitostéarate de polyglycérol, le palmitostéarate de polyglycol 1500 et 6000, le monolinoléate de glycérol ; les esters de glycérol éventuellement mono- ou diacétylés d'acides gras à longue chaîne tels que les monoglycérides monoacétylés ou diacétylés et leur mélange ; les glycérides hémisynthétiques.

On peut également ajouter un alcool gras dont la chaîne d'atomes de carbone est entre environ 16 et 22 atomes de carbone tel que par exemple, l'alcool myristylique, l'alcool palmitylique, l'alcool stéarylique.

Il est également possible de mettre en oeuvre des alcools gras polyoxyéthylénés résultant de la condensation avec l'oxyde d'éthylène à raison de 6 à 20 moles d'oxyde d'éthylène par mole, d'alcools gras linéaires ou ramifiés ayant de 10 à 20 atomes de carbone tels que, par exemple, l'alcool de coprah, le tridécanol ou l'alcool myristylique.

On peut citer également les cires telles que les cires microcristallines, la cire blanche, la cire de Carnauba, la paraffine.

On peut citer des sucres comme par exemple, du glucose, saccharose, fructose, galactose, ribose, maltose, sorbitol, mannitol, xylitol, lactitol, maltitol ; les sucres inversés : les sirops de glucose ainsi que les sucroglycérides dérivés d'huiles grasses telles que l'huile de coprah l'huile de palme, l'huile de palme hydrogénée et l'huile de soja hydrogénée ; les sucroesters d'acides gras tels que le monopalmitate de saccharose, le monodistéarate de saccharose et le distéarate de saccharose.

Comme exemples d'autres excipients, on peut mentionner les polysaccharides, et l'on peut citer, entre autres, les produits suivants et leurs mélanges :
- les amidons dérivés notamment de blé, de maïs, d'orge, de riz, de manioc ou de pomme de terre, natifs, prégélatinisés ou modifiés et plus particulièrement les amidons natifs de maïs riches en amylose, les amidons de maïs prégélatinisés, les amidons de maïs modifiés, les amidons de maïs cireux modifiés, les amidons de maïs cireux prégélatinisés, les amidons de maïs cireux modifiés en particulier l'amidon OSSA/octénylsuccinate sodique,
- les hydrolysats d'amidon,
- les dextrines et maltodextrines résultant de l'hydrolyse d'un amidon (blé, maïs) ou d'une fécule (pomme de terre) ainsi que les β-cyclodextrines,
- la cellulose, ses éthers, notamment la méthylcellulose, l'éthylcellulose, la méthyléthylcellulose, l'hydroxypropylcellulose ; ou ses esters, notamment la carboxyméthylcellulose ou la carboxyéthylcellulose éventuellement sous forme sodée,
- les gommes telles que la gomme de carraghénane, Kappa ou carraghénane Iota, la pectine, la gomme de guar, la gomme de caroube, et la gomme de xanthane, les alginates, la gomme arabique, la gomme d'acacia, l'agar-agar,

On choisit préférentiellement une maltodextrine ayant un degré d'hydrolyse mesuré par « dextrose équivalent » ou D.E inférieur à 20 et compris de préférence entre 5 et 19 et plus préférentiellement entre 6 et 15.

Comme autres excipients, on peut mentionner les farines notamment la farine de blé (native ou prégel) ; les fécules, plus particulièrement la fécule de pomme de terre, la fécule de Toloman, la fécule de maïs, la maïzena, le sagou ou le tapioca.

A titre d'excipients, il est également possible d'utiliser la gélatine (ayant de préférence, une force en gelée mesurée à l'aide d'un gélomètre de 100, 175 et 250 Bloom). Elle peut provenir indifféremment soit du traitement acide des peaux de porc et d'osséine, soit du traitement alcalin des peaux de bovins et d'osséïne.

Il est également possible d'additionner d'autres excipients tels que la silice ou bien par exemple un agent anti-oxydant comme notamment la vitamine E ou un agent émulsifiant notamment la lécithine.

Afin d'ajuster la puissance aromatique du mélange ou exhausser son gout, la mise en oeuvre d'éthylmaltol et/ou de propénylguétol peut être envisagée.

L'invention n'exclut pas l'addition d'une quantité supplémentaire de vanilline ou d'éthylvanilline.

Les compositions préférées de l'invention comprennent un sucre, de préférence le glucose, le saccharose, le fructose et/ou une dextrine ou maltodextrine : cette dernière ayant un DE avantageusement compris entre 6 et 15.

Le choix des excipients est effectué comme mentionné précédemment en fonction de l'application envisagée.

Le composé de l'invention peut être utilisé dans de nombreux domaines d'application, entre autres, dans le domaine alimentaire et pharmaceutique, et dans l'industrie de la parfumerie.

Un domaine d'application privilégié du composé de l'invention est celui de la biscuiterie et pâtisserie, et plus particulièrement :
- biscuiterie sèche : biscuits sucrés de type classique, petits beurre, galettes, casse-croûte, sablés,
- pâtisserie industrielle : boudoirs champagne, langues de chat, biscuits à la cuillère, pain de gênes, génoise, madeleines, quatre-quarts, cakes, pâtisserie aux amandes, petits fours.

Les éléments fondamentaux présents dans les mélanges destinés aux industries précitées sont les protéines (gluten) et l'amidon qui sont le plus souvent apportés par la farine de froment. Pour la préparation des divers types de biscuits et gâteaux, on ajoute à la farine, des ingrédients tels que saccharose, sel, oeufs, lait, corps gras, éventuellement levures chimiques (bicarbonate de sodium ou autres levures artificielles) ou levures biologiques et farines de céréales diverses etc...

L'incorporation du composé de vanilline et d'éthylvanilline selon l'invention est réalisée au cours de la fabrication, en fonction du produit souhaité et est conduite selon les techniques classiques du domaine considéré (cf. notamment J.L. KIGER et J.C. KIGER - Techniques Modernes de la Biscuiterie, Pâtisserie-Boulangerie industrielles et artisanales, DUNOD, Paris, 1968, Tome 2, pp. 231 et suivantes).

D'une manière préférentielle, le composé de l'invention est introduit dans les corps gras qui interviennent dans la préparation de la pâte.

A titre indicatif, on précisera que le composé de l'invention est introduit en une quantité de 0,005 à 0,2 g par kg de pâte.

Le composé de vanilline et d'éthylvanilline de l'invention est tout à fait adapté pour être utilisé dans le domaine de la chocolaterie et quelle que soit la forme de mise en oeuvre : chocolats en plaques, chocolats de couverture, fourrage pour chocolats.

Il peut être introduit au cours du conchage c'est-à-dire du malaxage de la pâte de cacao avec les différents ingrédients, notamment les arômes, soit après le conchage, par mise en oeuvre dans le beurre de cacao.

Dans ce domaine d'applications, le composé de vanilline et d'éthylvanilline de l'invention est utilisé selon le type de chocolat, à raison de 0,0005 g à 0,1 g pour 1 kg de produit fini : les teneurs les plus fortes se retrouvant dans le chocolat pour couverture.

Une autre utilisation du composé de l'invention est la fabrication des bonbons de tout genre : dragées, caramels, nougats, sucres cuits, bonbons fondants et autres.

La quantité du composé de l'invention introduite dépend du goût plus ou moins prononcé que l'on recherche. Ainsi, les doses d'utilisation du composé de l'invention peuvent varier entre 0,001 % et 0,2 %.

Le composé de l'invention convient bien à des utilisations dans l'industrie laitière et plus particulièrement dans les laits aromatisés et gélifiés, les entremets, les yaourts, les glaces et les crèmes glacées.

L'aromatisation se fait par simple addition du composé de l'invention, dans l'un des stades de mélange requis au cours de l'élaboration du produit.

Les teneurs dudit composé à mettre en oeuvre sont généralement faibles de l'ordre de 0,02 g pour 1 kg de produit fini.

Une autre application du composé de l'invention dans le domaine alimentaire est la préparation du sucre vanilliné c'est-à-dire l'imprégnation du sucre avec celles-ci, en une teneur de l'ordre de 7 g exprimée par rapport à 1 kg de produit fini.

Le composé de l'invention peut également intervenir dans différentes boissons et l'on peut citer, entre autres, la grenadine et les boissons chocolatées.

En particulier, il peut être mis en oeuvre dans les préparations pour boissons instantanées délivrées par les distributeurs automatiques de boissons, boissons aromatisées en poudre, chocolat en poudre ou bien dans les préparations instantanées sous forme de poudre destinées à la confection de desserts en tout genre, flans, pâtes à gâteaux, pancakes, après dilution à l'eau ou au lait.

Il est courant d'utiliser la vanilline pour la dénaturation du beurre. A cet effet, le composé de vanilline et d'éthylvanilline de l'invention peut être mis en oeuvre à raison de 6 g par tonne de beurre.

Un autre domaine d'application du composé de l'invention est l'alimentation animale, notamment pour la préparation de farines pour aliments des veaux et des porcs. La teneur préconisée est d'environ 0,2 g par kg de farine à aromatiser.

Le composé de l'invention peut trouver d'autres applications comme agent de masquage, pour l'industrie pharmaceutique (masquage de l'odeur de médicament) ou pour d'autres produits industriels (de type gomme, plastique, caoutchouc...).

Il convient tout à fait bien dans des domaines totalement différents tels que la cosmétique, l'industrie de la parfumerie ou la détergence.

Il peut être utilisé dans les cosmétiques tels que crèmes, laits, fards et autres produits et aussi, comme ingrédients parfumants, dans les compositions parfumantes, substances et produits parfumés.

Par "compositions parfumantes", on désigne des mélanges de divers ingrédients tels que solvants, supports solides ou liquides, fixateurs, composés odorants divers, etc..., dans lesquels est incorporé le composé de l'invention, lequel est utilisé pour procurer à divers types de produits finis, la fragrance recherchée.

Les bases pour parfum constituent des exemples préférés des compositions parfumantes dans lesquelles le composé de l'invention peut être avantageusement utilisé à raison d'une teneur de 0,1 % à 2,5 % en poids.

Les bases pour parfum peuvent servir à la préparation de nombreux produits parfumés tels que, par exemple, les eaux de toilettes, les parfums, les lotions après rasage ; les produits de toilette et d'hygiène tels que les gels de bain ou de douche, les produits déodorants ou antiperspirants, qu'ils soient sous forme de sticks ou de lotions, les talcs ou poudres de toute nature ; les produits pour les cheveux tels que les shampooings et les produits capillaires de tout type.

Un autre exemple de mise en oeuvre du composé de l'invention est le domaine de la savonnerie. Il peut être utilisé à une teneur de 0,3 % à 0,75 % de la masse totale à parfumer. Généralement, il est associé dans cette application, à du résinoïde de benjoin et de l'hyposulfite de sodium (2 %).

Le composé de vanilline et d'éthylvanilline selon l'invention peut trouver de nombreuses autres applications, notamment dans les désodorisants d'air ambiant ou tout produit d'entretien.

Dans les différentes applications précédemment mentionnées à titre illustratif, le composé de l'invention peut être introduit seul ou sous forme d'une composition le comprenant, associé à un ou plusieurs excipients dont certains exemples ont été donnés ci-dessus.

On donne ci-après des exemples illustrant la présente invention, sans caractère limitatif.

Dans les exemples, les pourcentages mentionnés sont exprimés en poids.

### Exemple 1

### Préparation du composé de l'invention.

Dans un flacon de 125 ml, on introduit 5,2 g de vanilline (VA) en poudre et 2,8 g d'éthylvanilline (EVA) en poudre, soit un rapport massique VA/EVA = 65/35.

Le mélange est homogénéisé par retournements du flacon.

Le flacon est ensuite placé dans une étuve à 70°C pendant 2 heures afin d'obtenir la fusion complète.

Le mélange fondu est alors coulé dans une coupelle en aluminium préchauffée dans l'étuve à 70°C ; le liquide est étalé de façon à former un film d'épaisseur homogène n'excédant pas 1 mm.

La coupelle est maintenue dans l'étuve dont la température est abaissée de 70 à 51 °C à raison de 1 °C/min puis un palier d'une heure minimum est observé à 51°C pour permettre la solidification totale du mélange VA-EVA.

La température de l'étuve est alors abaissée progressivement jusqu'à la température ambiante (environ 1 °C/min).

La plaque solide obtenue est broyée modérément à l'aide d'un granulateur à bras oscillant (calibreur Erweka type FGS) muni d'une toile de tamis de 1,6 mm d'ouverture.

Le composé de l'invention obtenu est sous forme de granulés.

### Caractéristiques physico-chimiques du composé de l'invention.

1. Le point de fusion du composé de l'invention est mesuré par analyse calorimétrique différentielle.
   La mesure est effectuée à l'aide d'un analyseur différentiel Mettler DSC822e dans les conditions suivantes :
   - préparation de l'échantillon à température ambiante : pesée et introduction dans un porte échantillon,
   - porte échantillon : capsule en aluminium sertie,
   - prise d'essai : 8,4 mg,
   - vitesse de montée en température : 2°C/min,
   - plage d'étude : 10 - 90°C.

   On pèse l'échantillon de composé qui est introduit dans la capsule qui est sertie puis placée dans l'appareil.
   On lance la programmation de température et l'on obtient le profil de fusion sur un thermogramme.
   La température de fusion est définie à partir d'un thermogramme réalisé dans les conditions opératoires précédentes.
   On retient la température onset : température correspondant à la pente maximale du pic de fusion.
   Le composé de l'invention a une température de fusion déterminée comme précédemment décrit (T onset) = 60°C.
2. Le spectre de diffraction des rayons X du composé de l'invention est déterminé à l'aide de l'appareil X'Pert Pro MPD PANalytical équipé d'un détecteur X' Celerator, dans les conditions suivantes :
   - Start Position [°2Th.] : 1,5124
   - End Position [°2Th.] : 49,9794
   - Step Size [°2Th.] : 0,0170
   - Scan Step Time [s] : 41,0051
   - Anode Materlal : Cu
   - K-Alpha1 [Å] : 1,54060
   - Generator Settings : 30 mA, 40 kV

Il est comparé à celui de la vanilline et de l'éthylvanilline.

La figure 1 représente trois courbes correspondant aux différents spectres de diffraction des rayons X du composé de l'invention, de la vanilline et de l'éthylvanilline.

Le spectre de diffraction des rayons X du composé de l'invention, présente plusieurs raies caractéristiques aux angles 2θ (en °) = 20,7 - 25,6 - 27,5 - 28,0 (mesurées par rapport à la raie du cuivre K-Alpha1 = 1,54060 Å), qui le différencie des spectres de la vanilline et de l'éthylvanilline.

Le composé de l'invention ne subit pas d'évolution après un stockage prolongé de 2 à 5 mois à température ambiante.

Ainsi, la figure 2 montre l'évolution du spectre de diffraction des rayons X du composé de l'invention, en fonction de la durée du stockage Elle représente trois courbes correspondant aux différents spectres de diffraction des rayons X du composé de l'invention obtenu à l'instant t = 0, puis, après un stockage de 2 mois et de 5 mois.

Les 3 courbes obtenues sont normalement superposées. Afin de pouvoir mieux les différencier, deux des 3 courbes de la figure 2, ont une ligne de base volontairement décalée par rapport à la ligne de base de référence qui est le spectre de diffraction des rayons X à l'instant t = 0. La courbe correspondant au spectre de diffraction des rayons X obtenue après un stockage de 2 mois est décalée de 5 000 coups/s et celle obtenue après un stockage de 5 mois est décalée de 10 000 coups/s.

La figure 2 met en évidence qu'il n'y a pas d'évolution du composé de l'invention après un stockage prolongé.

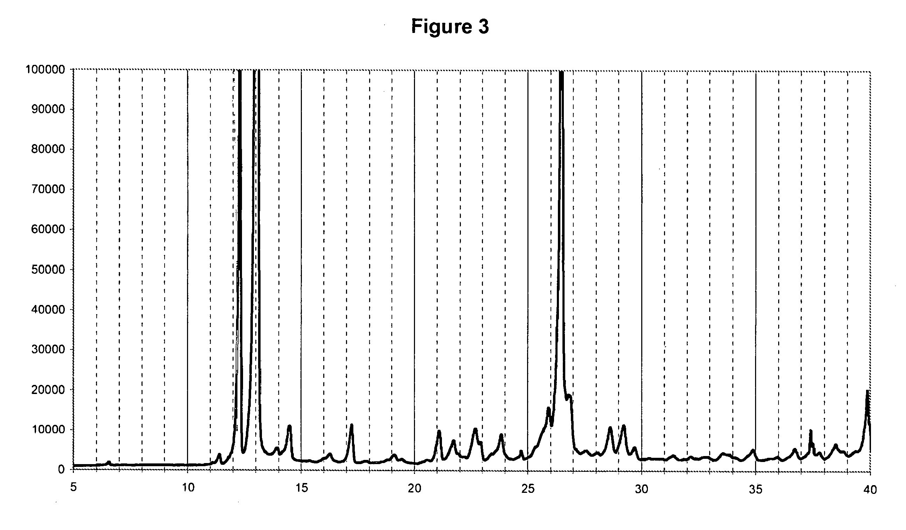

La figure 3 représente à titre de comparaison, le spectre de diffraction des rayons X d'un mélange à sec des 2 poudres de vanilline et d'éthylvanilline dans un ratio molaire de 2.

Les conditions de mesure sont celles mentionnées ci-dessus.

Le spectre de diffraction des rayons X du mélange ne présente pas les raies caractéristiques du composé de l'invention. 3. Pour ce qui est de l'hygroscopicité, elle est quantifiée par l'intermédiaire de la prise de poids d'un échantillon du composé de l'invention placé en couche mince (1 à 2 mm d'épaisseur) dans une enceinte climatique à 40°C et sous air à 80 % d'humidité relative pendant 1 heure.

Le composé de l'invention, n'adsorbe, après maintien pendant 1 heure, à 40°C et sous air à 80 % d'humidité relative, que 0,27 % d'eau en poids ; cette prise de masse est totalement réversible par retour à 25°C et sous 40 % d'humidité relative.

Les granulés obtenus, conservés un mois à 22°C dans un flacon bouché, présentent toujours une bonne coulabilité.

A titre de comparaison, un mélange des 2 poudres de vanilline et d'éthylvanilline, conservé dans les mêmes conditions est totalement pris en masse après une semaine et ceci quel que soit le rapport massique VA/EVA compris entre 2/98 et 98/2.

### Exemple 2

Dans un flacon de 125 ml, on introduit 4,9 g d'éthanol absolu, 5,2 g de vanilline et 2,8 g d'éthylvanilline, soit un rapport massique VA/EVA = 65/35.

Le flacon est agité à l'aide d'un roule-flacon et est maintenu à 25°C jusqu'à dissolution complète des 2 produits (environ 2 heures).

Le flacon est alors placé dans un réfrigérateur à 3°C pendant une dizaine d'heures.

On observe l'apparition d'une phase solide blanche qui est rapidement séparée du liquide par filtration.

Le solide ainsi obtenu est séché sous vide (100 mm de mercure, soit 1,33×10^{a} Pa), d'abord à 20°C pendant une heure puis la température est augmentée lentement à raison de 1°C/min jusqu'à 52°C.

Le séchage sous vide (100 mm de mercure, soit 1,33×10⁴ Pa) est poursuivi à 52°C pendant une heure.

Le produit sec a un point de fusion de 61°C mesuré par analyse calorimétrique différentielle.

Son spectre de diffraction des rayons X présente les raies caractéristiques qui le différencient de la vanilline et de l'éthylvanilline.

### Exemple 3

Dans un flacon de 125 ml, on introduit 8,5 g de vanilline, 4,6 g d'éthylvanilline et 1,0 g d'eau déminéralisée.

Ce flacon est placé dans une étuve à 62°C pendant 2 heures de façon à obtenir une seule phase liquide homogène.

Ce liquide est coulé dans une coupelle en aluminium et est étalé de façon à former un film d'épaisseur homogène.

La coupelle est alors placée dans un réfrigérateur à 3°C pendant une dizaine d'heures.

On observe alors une prise en masse totale de tout le produit.

Après retour à température ambiante, le produit reste un solide qui est broyé modérément à l'aide d'un granulateur à bras oscillant (calibreur Erweka type FGS) muni d'une toile de tamis de 1,6 mm d'ouverture.

Le composé de l'invention obtenu sous forme de granulés est séché sous vide (100 mm de mercure, soit 1,33×10⁴ Pa), d'abord à 20°C pendant une heure puis la température est augmentée lentement à raison de 1°C/min jusqu'à 52°C.

Le séchage sous vide (100 mm de mercure, soit 1,33×10⁴ Pa) est poursuivi à 52°C pendant une heure.

Le produit sec a un point de fusion de 60°C mesuré par analyse calorimétrique différentielle.

Son spectre de diffraction des rayons X présente les raies caractéristiques qui le différencient de la vanilline et de l'éthylvanilline.

### Exemples 4 à 6

Dans les exemples 4 à 6, on prépare des granulés du composé de l'invention (exemple 4) et des compositions les comprenant (exemples 5 et 6).

On observe leur comportement au stockage en comparaison avec de la vanilline (exemple comparatif A), de l'éthylvanilline (exemple comparatif B) et d'un mélange à sec de vanilline et d'éthylvanilline (exemple comparatif C).

### Exemple 4

Dans un réacteur agité équipé d'un chauffage par double enveloppe, on introduit 350 g de vanilline en poudre et 188,5 g d'éthylvanilline en poudre, soit un rapport molaire de 2 entre la vanilline et l'éthylvanilline. L'humidité de ces poudres est de 0,1% ± 0,02% en poids.

Ce mélange est porté à 70°C sous agitation. On obtient ainsi une phase liquide homogène.

Le mélange fondu est coulé sur une plaque en inox maintenue à 50°C de façon à y former un film mince d'environ 1 mm d'épaisseur. La cristallisation est complète en une dizaine de minutes.

La plaque solide ainsi formée se décolle aisément de l'inox ; elle est laissée à température ambiante jusqu'à refroidissement complet.

Cette plaque est ensuite concassée grossièrement pour pouvoir alimenter un granulateur à bras oscillant (calibreur Erweka type FGS) muni d'une toile de tamis de 1,0 mm d'ouverture. Le produit y est broyé modérément pour conduire à des granulés dont la taille varie de 0,1 à 1,0 mm.

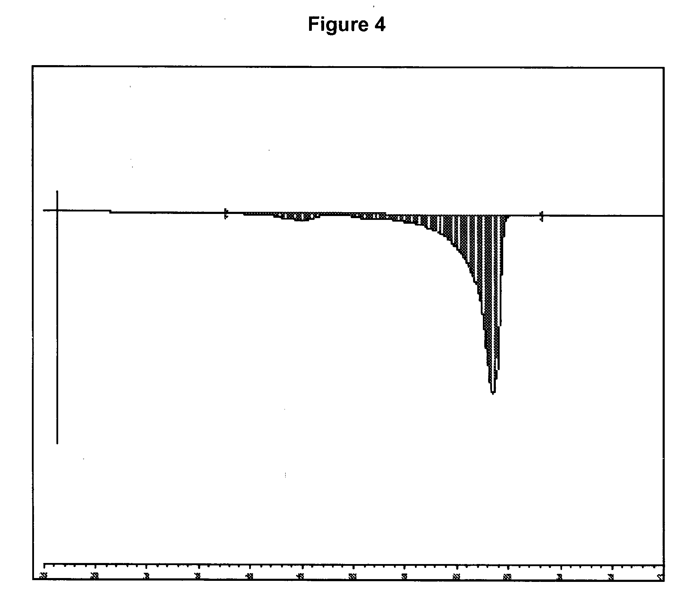

Les granulés ainsi obtenus ont un point de fusion de 59,8°C mesuré par analyse calorimétrique différentielle (T onset) et déterminé à partir du thermogramme représenté à la figure 4.

Le thermogramme est un graphe qui représente la puissance thermique fournie à l'échantillon (exprimée en w/g) en fonction de la température entre 20°C et 90°C.

L'intégrale de la courbe obtenue permet de déterminée l'enthalpie de fusion soit 129,5 J/g.

Leur spectre de diffraction des rayons X présente les raies caractéristiques aux angles 2θ (°) = 20,7 - 25,6 - 27,5 - 28,0 qui le différencient des spectres de la vanilline et de l'éthylvanilline, comme illustré sur la figure 1.

### Exemple 5

Les granulés préparés selon l'exemple 4, peuvent être mélangés à sec avec un excipient, par exemple à 50/50 en poids, ce qui améliore encore leur propriété de coulabilité.

Dans cet exemple, on prépare une composition comprenant 50 % en poids des granulés préparés selon l'exemple 4 et 50 % en poids d'un excipient, le saccharose.

L'opération de mélangeage d'environ 5 min est effectuée à température ambiante dans un mélangeur WAM à socs de charrue (plough mixer).

### Exemple 6

Dans cet exemple, on prépare une composition comprenant 50 % en poids des granulés préparés selon l'exemple 4 et 50 % en poids d'une maltodextrine ayant un DE de 6 (Roquette Glucidex IT6).

L'opération de mélangeage est effectuée comme décrite à l'exemple 5.

### Exemples comparatifs A à C

Ces exemples font intervenir respectivement la vanilline, l'éthylvanilline et le mélange à sec des poudres de vanilline et d'éthylvanilline dans un ratio molaire de 2 effectué dans un mélangeur comme dans l'exemple 4.

Les propriétés de coulabilité et d'aptitude au mottage du composé de l'invention et des compositions le mettant en oeuvre sont comparées à celle de la poudre de vanilline, de la poudre d'éthylvanilline et d'un simple mélange à sec de ces 2 poudres.

La coulabilité des poudres est une notion technique bien connue de l'homme du métier. Pour plus de détails, on peut se reporter notamment à l'ouvrage "Standard shear testing technique for particulate solids using the Jenike shear cell", publié par "The Institution of Chemicals Engineers", 1989 (ISBN : 0 85295 232 5).

La mesure de l'incise de coulabilité est effectuée de la manière qui suit.

La coulabilité des poudres est mesurée par cisaillement d'un échantillon dans une cellule annulaire (commercialisée par D. Schulze, Allemagne).

Le précisaillement des poudres est effectué sous une contrainte normale de 5200 Pa.

Les points de cisaillement nécessaires au tracé du lieu d'écoulement de l'échantillon sont obtenus pour 4 contraintes normales inférieures à la contrainte du précisaillement, typiquement 480 Pa, 850 Pa, 2 050 Pa et 3020 Pa.

A partir des cercles de Mohr dans le diagramme "contrainte de cisaillement en fonction des contraintes normales", on détermine sur le lieu d'écoulement 2 contraintes qui caractérisent l'échantillon :
- la contrainte normale dans la direction principale ; elle est donnée par l'extrémité du grand cercle de Mohr qui passe par le point de précisaillement,
- la force de cohésion ; elle est donnée par l'extrémité du petit cercle de Mohr qui est tangent au lieu d'écoulement et passe par l'origine.

Le rapport entre la contrainte normale dans la direction principale et la force de cohésion est un nombre adimensionnel appelé "i, indice de coulabilité".

Ces mesures sont réalisées immédiatement après remplissage de la cellule annulaire, on obtient ainsi l'indice de coulabilité instantané.

Une autre série de mesures est réalisée avec une cellule qui a été stockée pendant 24 heures à 40°C et 80% d'humidité relative sous une contrainte normale de 2 400 Pa.

On obtient ainsi l'indice de mottage.

Les résultats reportés dans le tableau (I) permettent de comparer les indices de coulabilité instantanée et les indices de mottage de la poudre de vanilline (exemple comparatif A), de la poudre d'éthylvanilline (exemple comparatif B), d'un simple mélange à sec de ces 2 poudres (exemple comparatif C), des granulés obtenus selon le procédé de l'invention (exemple 4), des granulés obtenus selon le procédé de l'invention et mélangés à 50/50 en poids avec du saccharose (exemple 5), des granulés obtenus selon le procédé de l'invention et mélangés à 50/50 en poids avec une maltodextrine (exemple 6).

**Tableau (I)**

| Référence | Nature du produit | Indice de coulabilité instantanée | Indice de mottage après stockage |
|---|---|---|---|
| Exemple comparatif A | Poudre de vanilline | 5,6 | 0,66 |
| Exemple comparatif B | Poudre d'éthylvanilline | 6,5 | 0,61 |
| Exemple comparatif C | Mélange des poudres de vanilline et d'éthylvanilline ratio molaire = 2 | 18 | 0,03 |
| Exemple 4 | Granulés de l'invention décrits dans l'exemple 4 | 22 | 0,13 |
| Exemple 5 | Composition comprenant les granulés de l'exemple 4 et du saccharose | 20 | 0,18 |
| Exemple 6 | Composition comprenant les granulés de l'exemple 4 et une maltodextrine | 34 | 0,73 |

On constate que les granulés obtenus selon le procédé de l'invention ont un indice de mottage après stockage sous contrainte très supérieur à celui d'un simple mélange à sec des poudres de vanilline et d'éthylvanilline.

En mélange à 50/50 en poids avec une maltodextrine, ces granulés ont un indice de mottage comparable à celui des poudres de vanilline pure ou d'éthylvanilline pure.

## Revendications

1. Composé de vanilline et d'éthylvanilline dans un ratio molaire vanilline/éthylvanilline de 2 ayant un point de fusion de 60°C ± 2°C et présentant un spectre de diffraction des rayons X avec plusieurs raies caractéristiques aux angles 2θ (en °) = 20,7 - 25,6 - 27,5 - 28,0 (mesurées par rapport à la raie du cuivre K-Alpha1 = 1,54060 Å).

2. Composé selon la revendication 1 **caractérisé par le fait qu'**il adsorbe moins de 0,5% en poids d'eau, de préférence entre 0,1 et 0,3 % en poids d'eau et **par le fait qu'**il reste parfaitement solide à 40°C sous air à 80 % d'humidité relative.

3. Composé selon la revendication 1 ou 2 **caractérisé par le fait qu'**il présente des propriétés de coulabilité améliorées et l'absence de mottage après stockage à 22°C, pendant 1 mois.

4. Procédé de préparation du composé décrit dans l'une des revendications 1 à 3 **caractérisé par le fait qu'**il comprend la co-cristallisation de vanilline et d'éthylvanilline mises en oeuvre dans un ratio molaire vanilline/éthylvanilline de 2, en milieu fondu ou en solution dans un solvant les solubilisant.

5. Procédé selon la revendication 4 **caractérisé par le fait que** l'on solubilise par chauffage et sous agitation, le mélange de vanilline et d'éthylvanilline dans le solvant, que l'on refroidit l'ensemble et sépare les cristaux du composé obtenu.

6. Procédé selon la revendication 5 **caractérisé par le fait que** le solvant organique est l'eau et/ou un solvant organique, polaire, protique ou aprotique.

7. Procédé selon l'une des revendications 5 et 6 **caractérisé par le fait que** le solvant est choisi parmi :
- l'eau,
- les alcools, de préférence, aliphatiques ou arylaliphatiques et plus préférentiellement, le méthanol, l'éthanol, le propanol, l'isopropanol, le butanol, l'alcool β-phényléthylique, l'éthylèneglycol, le diéthylèneglycol, le propylèneglycol, le glycérol,
- les éther-oxydes de préférence, aliphatiques, et, plus particulièrement, le diéthyléther, le dipropyléther, le diisopropyléther, le dibutyléther, le méthyltertiobutyléther, l'éthyltertiobutyléther, le ditertiobutyléther, le diméthyléther de l'éthylèneglycol, le diméthyléther du diéthylèneglycol,
- les esters d'alkyle ou d'arylalkyle d'acides carboxyliques, aliphatiques, cycloaliphatiques ou aromatiques, et plus préférentiellement, l'acétate d'éthyle, l'acétate de butyle, le salicylate de benzyle, le laurate de méthyle, le benzoate de méthyle, le citrate d'éthyle, le triacétylglycérol ou triacétine, ester du glycérol et de l'acide acétique.

8. Procédé selon l'une des revendications 5 à 7 **caractérisé par le fait que** le solvant est l'eau, l'éthanol, le propylèneglycol, la triacétine et leurs mélanges.

9. Procédé selon l'une des revendications 5 à 8 **caractérisé par le fait que** la quantité de solvant mis en oeuvre, exprimée en poids par rapport au poids de matières sèches (vanilline + éthylvanilline) est de 5 à 60 %.

10. Procédé selon la revendication 4 **caractérisé par le fait que** l'on effectue la fusion du mélange vanilline et éthylvanilline mis en oeuvre dans un ratio molaire de 2 puis le refroidissement du mélange fondu en abaissant la température à 50°C ± 1, puis l'on maintient cette température jusqu'à solidification totale du mélange.

11. Procédé selon la revendication 10 **caractérisé par le fait que** l'on charge la vanilline et l'éthylvanilline mis en oeuvre dans un ratio molaire de 2, séparément ou en mélange et l'on porte le mélange à une température qui est choisie entre 60°C et 90°C et qui se situe de préférence entre 70°C et 80°C.

12. Procédé selon la revendication 11 **caractérisé par le fait que** la préparation de ce mélange fondu est faite sous atmosphère de gaz inertes qui est préférentiellement de l'azote.

13. Procédé selon l'une des revendications 10 à 12 **caractérisé par le fait que** l'on effectue le refroidissement du mélange fondu en l'absence d'agitation.

14. Procédé selon l'une des revendications 4 à 13 **caractérisé par le fait que** le composé obtenu est mis en forme selon une technique de broyage.

15. Procédé selon l'une des revendications 10 à 12 **caractérisé par le fait que** le mélange fondu est mis en forme selon une technique d'écaillage.

16. Composé de vanilline et d'éthylvanilline **caractérisé par le fait qu'**il est obtenu par fusion d'un mélange de vanilline et d'éthylvanilline mises en oeuvre dans un ratio molaire de 2 à une température choisie entre 60°C et 90°C et de préférence entre 70°C et 80°C suivie par un refroidissement du mélange fondu en abaissant la température à 50°C ± 1, puis maintien de cette température jusqu'à solidification totale du mélange.

17. Composition comprenant au moins un composé de vanilline et d'éthylvanilline décrit dans l'une des revendications 1 à 3, 16 et au moins un excipient choisi parmi les corps gras ; les alcools gras ; les sucres ; les polysaccharides la silice ; la vanilline et l'éthylvanilline.

18. Composition selon la revendication 17 **caractérisée par le fait que** l'excipient est choisi parmi :
- les sucres de préférence le glucose, saccharose, fructose, galactose, ribose, maltose, sorbitol, mannitol, xylitol, lactitol, maltitol ; les sucres inversés : les sirops de glucose ainsi que les sucroglycérides dérivés d'huiles grasses de préférence l'huile de coprah l'huile de palme, l'huile de palme hydrogénée et l'huile de soja hydrogénée ; les sucroesters d'acides gras de préférence le monopalmitate de saccharose, le monodistéarate de saccharose et le distéarate de saccharose,
- les amidons dérivés notamment de blé, de maïs, d'orge, de riz, de manioc ou de pomme de terre, natifs, prégélatinisés ou modifiés et plus particulièrement les amidons natifs de maïs riches en amylose, les amidons de maïs prégélatinisés, les amidons de maïs modifiés, les amidons de maïs cireux modifiés, les amidons de maïs cireux prégélatinisés, les amidons de maïs cireux modifiés en particulier l'amidon OSSA/octénylsuccinate sodique,
- les hydrolysats d'amidon,
- les dextrines et maltodextrines résultant de l'hydrolyse d'un amidon (blé, maïs) ou d'une fécule (pomme de terre) ainsi que les β-cyclodextrines, de préférence les maltodextrines ayant un D.E inférieur à 20, compris de préférence entre 5 et 19 et plus préférentiellement entre 6 et 15,
- la cellulose, ses éthers, notamment la méthylcellulose, l'éthylcellulose, la méthyléthylcellulose, l'hydroxypropylcellulose; ou ses esters, notamment la carboxyméthylcellulose ou la carboxyéthylcellulose éventuellement sous forme sodée,
- les gommes telles que la gomme de carraghénane, Kappa ou carraghénane Iota, la pectine, la gomme de guar, la gomme de caroube, et la gomme de xanthane, les alginates, la gomme arabique, la gomme d'acacia, l'agar-agar,
- les farines de préférence la farine de blé (native ou prégel) ; les fécules, de préférence la fécule de pomme de terre,
- la gélatine,
- la silice,
- un agent anti-oxydant de préférence la vitamine E,
- un agent émulsifiant de préférence la lécithine,
- de la vanilline ou de l'éthylvanilline.

19. Composition selon l'une des revendications 17 et 18 **caractérisée par le fait qu'**elle comprend de 0,1 à 90 % en poids d'excipient(s), de préférence de 20 à 60 % en poids d'excipient(s).

20. Application du composé décrit dans l'une des revendications 1 à 3, 16 ou de la composition décrite dans l'une des revendications 17 à 19 comme arôme dans le domaine de l'alimentation humaine et animale, la pharmacie, et comme parfum, dans l'industrie des cosmétiques, de la parfumerie et de la détergence.

21. Application selon la revendication 20 **caractérisée par le fait que** l'on met en oeuvre le composé ou la composition de l'invention, au cours de la fabrication d'une pâte, de préférence dans un corps gras, dans le domaine de la biscuiterie sèche et de la pâtisserie industrielle ; dans le domaine de la chocolaterie notamment pour la préparation des chocolats en plaques, des chocolats de couverture ou du fourrage pour chocolats ; au cours de la fabrication des bonbons de tout genre : dragées, caramels, nougats, sucres cuits, bonbons fondants et autres ; dans l'industrie laitière et plus particulièrement dans les laits aromatisés et gélifiés, les entremets, les yaourts, les glaces et les crèmes glacées ; dans la préparation du sucre vanilliné, par imprégnation du sucre avec celle-ci ; dans la préparation de différentes boissons, de préférence, la grenadine et les boissons chocolatées ; dans les préparations de boissons instantanées telles que boissons aromatisées en poudre, chocolat en poudre ou bien dans les préparations instantanées sous forme de poudre destinées à la confection de desserts en tout genre ; pour la dénaturation du beurre.

22. Application selon la revendication 20 **caractérisée par le fait que** l'on met en oeuvre, le composé ou la composition de l'invention dans l'alimentation animale, notamment pour la préparation de farines.

23. Application selon la revendication 20 **caractérisée par le fait que** l'on met en oeuvre le composé ou la composition de l'invention comme agent de masquage d'odeur, notamment dans l'industrie pharmaceutique ; dans le domaine de la cosmétique pour la préparation de crèmes, laits et fards et autres produits, comme base parfumante dans la parfumerie et dans le domaine de la détergence, notamment en savonnerie.

## Patentansprüche

1. Verbindung aus Vanillin und Ethylvanillin in einem Vanillin/Ethylvanillin-Molverhältnis von 2, die einen Schmelzpunkt von 60 °C ± 2 °C hat und die ein Röntgendiffraktionsspektrum mit mehreren charakteristischen Linien mit den Winkeln 2Θ (in °) = 20,7 - 25,6 - 27,5 - 28,0 aufweist (gemessen bezogen auf die Kupferlinie K-Alpha1 = 1,54060 λ).

2. Verbindung nach Anspruch 1, **dadurch gekennzeichnet, dass** sie weniger als 0,5 Gew.-%, bevorzugt zwischen 0,1 und 0,3 Gew.-% Wasser adsorbiert, und dadurch, dass sie bei 40 °C unter Luft bei 80 % relativer Feuchtigkeit vollkommen fest bleibt.

3. Verbindung nach Anspruch 1 oder 2, **dadurch gekennzeichnet, dass** sie verbesserte Fließeigenschaften und nach einmonatiger Lagerung bei 22 °C Abwesenheit von Klumpenbildung aufweist.

4. Verfahren zur Herstellung der in einem der Ansprüche 1 bis 3 beschriebenen Verbindung, **dadurch gekennzeichnet, dass** sie die gleichzeitige Kristallisation von Vanillin und Ethylvanillin, welche in einem Vanillin/Ethylvanillin-Molverhälnis von 2 eingesetzt werden, in geschmolzenem Medium oder in Lösung in einem sie solubilisierenden Lösemittel umfasst.

5. Verfahren nach Anspruch 4, **dadurch gekennzeichnet, dass** das Gemisch aus Vanillin und Ethylvanillin in dem Lösemittel durch Erhitzen und unter Schütteln solubilisiert wird, dass das Ganze abgekühlt wird und die Kristalle der erhaltenen Verbindung abgetrennt werden.

6. Verfahren nach Anspruch 5, **dadurch gekennzeichnet, dass** das Lösemittel Wasser und/oder ein organisches, polares, protisches oder aprotisches Lösemittel ist.

7. Verfahren nach einem der Ansprüche 5 und 6, **dadurch gekennzeichnet, dass** das Lösemittel ausgewählt ist aus:
- Wasser,
- Alkoholen, bevorzugt aliphatischen oder arylaliphatischen Alkoholen und stärker bevorzugt Methanol, Ethanol, Propanol, Isopropanol, Butanol, β-Phenylethylalkohol, Ethylenglykol, Diethylenglykol, Propylenglykol, Glycerol,
- Etheroxiden, bevorzugt aliphatischen Etheroxiden und insbesondere Diethylether, Dipropylether, Diisopropylether, Dibutylether, Methyltertiobutylether, Ethyltertiobutylether, Ditertiobutylether, Ethylenglykoldimethylether, Diethylenglykoldimethylether,
- aliphatischen, cycloaliphatischen oder aromatischen Alkyl- oder Arylalkylestern von Carbonsäuren und insbesondere Ethylacetat, Butylacetat, Benzylsalicylat, Methyllaurat, Methylbenzoat, Ethylcitrat, Triacetylglycerol oder Triacetin, Glycerolester und Essigsäureester.

8. Verfahren nach einem der Ansprüche 5, bis 7, **dadurch gekennzeichnet, dass** das Lösemittel Wasser, Ethanol, Propylenglykol, Triacetin und deren Gemische ist.

9. Verfahren nach einem der Ansprüche 5 bis 8, **dadurch gekennzeichnet, dass** die eingesetzte Menge an Lösemittel, ausgedrückt in Gewicht bezogen auf das Trockengewicht (Vanillin + Ethylvanillin) bei 5 bis 60 % liegt.

10. Verfahren nach Anspruch 4, **dadurch gekennzeichnet, dass** das Schmelzen des in einem Molverhältnis von 2 eingesetzten Gemisches aus Vanillin und Ethylvanillin, dann das Abkühlen des geschmolzenen Gemischs durchgeführt wird, indem die Temperatur auf 50 °C ± 1 gesenkt und dann diese Temperatur bis zur vollständigen Verfestigung des Gemischs aufrecht erhalten wird.

11. Verfahren nach Anspruch 10, **dadurch gekennzeichnet, dass** das in einem Molverhältnis von 2 eingesetzte Vanillin und Ethylvanillin, getrennt oder als Gemisch, geladen wird und dass das Gemisch auf eine Temperatur gebracht wird, die zwischen 60 °C und 90 °C ausgewählt ist und die bevorzugt zwischen 70 °C und 80 °C liegt.

12. Verfahren nach Anspruch 11, **dadurch gekennzeichnet, dass** die Herstellung dieses geschmolzenen Gemischs unter einer Atmosphäre aus inerten Gasen erfolgt, bei dem es sich bevorzugt um Stickstoff handelt.

13. Verfahren nach einem der Ansprüche 10 bis 12, **dadurch gekennzeichnet, dass** das Abkühlen des geschmolzenen Gemischs in Abwesenheit von Schütteln ausgeführt wird.

14. Verfahren nach einem der Ansprüche 4 bis 13, **dadurch gekennzeichnet, dass** die erhaltene Verbindung durch eine Mahltechnik gebildet wird.

15. Verfahren nach einem der Ansprüche 10 bis 12, **dadurch gekennzeichnet, dass** das geschmolzene Gemisch durch eine flockenbildende Technik gebildet wird.

16. Verbindung aus Vanillin und Ethylvanillin, **dadurch gekennzeichnet, dass** sie durch Schmelzen eines Gemischs aus Vanillin und Ethylvanillin, welche in einem Molverhältnis von 2 eingesetzt werden, bei einer Temperatur, die zwischen 60 °C und 90 °C und bevorzugt zwischen 70 °C und 80 °C ausgewählt ist, gefolgt von einer Abkühlung des geschmolzenen Gemischs durch Senken der Temperatur auf 50 °C ± 1, dann Aufrechterhalten dieser Temperatur bis zur vollständigen Verfestigung des Gemischs, erhalten wird.

17. Zusammensetzung, umfassend mindestens eine in einem der Ansprüche 1 bis 3, 16 beschriebene Verbindung aus Vanillin und Ethylvanillin und mindestens einem Exzipienten, ausgewählt aus Fetten; Fettalkoholen; Zuckern; Polysacchariden; Kieselerde; Vanillin und Ethylvanillin.

18. Zusammensetzung nach Anspruch 17, **dadurch gekennzeichnet, dass** der Exzipient ausgewählt ist aus:
- Zuckern, bevorzugt Glucose, Saccharose, Fructose, Galactose, Ribose, Maltose, Sorbitol, Mannitol, Xylitol, Lactitol, Maltitol; Invertzuckern: Glucosesirupen sowie von Fettölen abgeleitete Zuckerglyceriden, bevorzugt Kokosöl, Palmöl, hydrogeniertes Palmöl und hydrogeniertes Sojaöl; Saccharoseestern von Fettsäuren, bevorzugt Saccharosemonopalmitat, Saccharosemonodistearat und Saccharosedistearat,
- nativen, vorgelatinierten oder modifizierten Stärken, insbesondere abgeleitet von Weizen, Mais, Gerste, Reis, Maniok oder Kartoffel, und insbesondere amylosereichen nativen Maisstärken, vorgelatinisierten Maisstärke, modifizierten Maisstärken, modifizierten Maiswachsstärken, vorgelatinisierten Wachsmaisstärken, modifizierten Wachsmaisstärken, insbesondere die Stärke OSSA/Natriumoctenylsuccinat,
- Stärkehydrolysaten,
- Dextrinen und Maltodextrinen, die aus der Hydrolyse einer Weizen- oder Maisstärke oder einer Kartoffelstärke resultieren sowie β-Cyclodextrinen, bevorzugt Maltodextrinen, die einen DE-Wert kleiner als 20, bevorzugt im Bereich zwischen 5 und 19 und stärker bevorzugt zwischen 6 und 15 haben,
- Cellulose, ihren Ethern, insbesondere Methylcellulose, Ethylcellulose, Methylethylcellulose, Hydroxypropylcellulose; oder ihren Estern, insbesondere Carboxymethylcellulose oder Carboxyethylcellulose, gegebenenfalls in Natriumform,
- Gummis, wie etwa Carrageen, Kappa-Carrageen oder Iota-Carrageen, Pektin, Guargummi, Johannisbrotkernmehl, Xanthangummi, Alginaten, Gummi arabicum, Akaziengummi, Agar-Agar,
- Mehlen, bevorzugt Weizenmehl (nativ oder vorgelatinisiert); Stärken, bevorzugt Kartoffelstärke,
- Gelatine,
- Kieselerde,
- einem Antioxidationsmittel, bevorzugt Vitamin E,
- einem Emulgator, bevorzugt Lecithin,
- Vanillin oder Ethylvanillin.

19. Zusammensetzung nach einem der Ansprüche 17 und 18, **dadurch gekennzeichnet, dass** sie 0,1 bis 90 Ges.-% Exzipient(en), bevorzugt 20 bis 60 Gew.-% Exzipient(en) umfasst.

20. Anwendung der in einem der Ansprüche 1 bis 3, 16 beschriebenen Verbindung oder der in einem der Ansprüche 17 bis 19 beschriebenen Zusammensetzung als Aroma im Bereich der menschlichen oder tierischen Ernährung, der Pharmazie und als Parfum in der Kosmetik-, Parfümerie- und Reinigungsmittelindustrie.

21. Anwendung nach Anspruch 20, **dadurch gekennzeichnet, dass** die Verbindung oder die Zusammensetzung der Erfindung während der Herstellung einer Masse, bevorzugt in einem Fett, im Bereich der Trockenkeksherstellung und der industriellen Patisserie; im Bereich der Schokoladenherstellung, insbesondere zur Herstellung von Schokoladentafeln, Kuvertüreschokoladen oder Füllungen für Schokoladen; bei der Herstellung von Bonbons jeder Art: Dragees, Karamellbonbons, Nougatbonbons, Hartkaramellen, Fondantbonbons und anderen; in der milchverarbeitenden Industrie und insbesondere in aromatisierten Milch- und Puddingprodukten, Süßspeisen, Joghurts, Gefrorenem und Eiscremen; bei der Herstellung von Vanillinzucker, durch Imprägnieren des Zuckers mit derselben; bei der Herstellung verschiedener Getränke, bevorzugt Grenadine und schokoladehaltigen Getränken; bei den Herstellungen von Instantgetränken, wie etwa aromatisierten Getränken in Pulverform, Kakaopulver mit Zucker oder auch bei den Instantzubereitungen in Pulverform, die zur Zubereitung von Desserts jeder Art bestimmt sind; zur Denaturierung von Butter, eingesetzt wird.

22. Anwendung nach Anspruch 20, **dadurch gekennzeichnet, dass** die Verbindung oder die Zusammensetzung der Erfindung in der tierischen Ernährung, insbesondere zur Herstellung von Mehlen, eingesetzt wird.

23. Anwendung nach Anspruch 20, **dadurch gekennzeichnet, dass** die Verbindung oder die Zusammensetzung der Erfindung als Geruchsmaskierungsmittel, insbesondere in der pharmazeutischen Industrie; im Bereich der Kosmetik zur Herstellung von Cremen, Milch und Schminken und anderen Produkten, als Parfumgrundstoff in der Parfümerie und im Bereich der Reinigungsmittel, insbesondere der Seifenherstellung, eingesetzt wird.

## Claims

1. Compound of vanillin and ethylvanillin in a vanillin/ethylvanillin molar ratio of 2 having a melting point of 60°C ± 2°C and having an X-ray diffraction spectrum with several characteristic lines at angles 2θ (in °) = 20.7 - 25.6 - 27.5 - 28.0 (measured relative to the line of copper K-Alpha1 = 1.54060 Å).

2. Compound according to Claim 1, **characterized in that** it adsorbs less than 0.5 wt.% of water, preferably between 0.1 and 0.3 wt.% of water and **in that** it remains perfectly solid at 40°C in air at 80% relative humidity.

3. Compound according to Claim 1 or 2, **characterized in that** it displays improved flowability and absence of lumpiness after storage at 22°C for 1 month.

4. Method of preparation of the compound described in one of Claims 1 to 3, **characterized in that** it comprises the co-crystallization of vanillin and ethylvanillin used in a vanillin/ethylvanillin molar ratio of 2, in a molten medium or in solution in a solvent that dissolves them.

5. Method according to Claim 4, **characterized in that** the mixture of vanillin and ethylvanillin is dissolved in the solvent with heating and stirring, the whole is cooled and the crystals of the compound obtained are separated.

6. Method according to Claim 5, **characterized in that** the solvent is water and/or a protic or aprotic polar organic solvent.

7. Method according to either of Claims 5 and 6, **characterized in that** the solvent is selected from:
- water,
- alcohols, preferably aliphatic or arylaliphatic and more preferably methanol, ethanol, propanol, isopropanol, butanol, β-phenylethyl alcohol, ethylene glycol, diethylene glycol, propylene glycol, glycerol,
- ether-oxides, preferably aliphatic, and more particularly diethyl ether, dipropyl ether, diisopropyl ether, dibutyl ether, methyl tert-butyl ether, ethyl tert-butyl ether, ditert-butyl ether, ethylene glycol dimethyl ether, diethylene glycol dimethyl ether,
- alkyl or aralkyl esters of aliphatic, cycloaliphatic or aromatic carboxylic acids, and more preferably ethyl acetate, butyl acetate, benzyl salicylate, methyl laurate, methyl benzoate, ethyl citrate, triacetylglycerol or triacetin, ester of glycerol and of acetic acid.

8. Method according to one of Claims 5 to 7, **characterized in that** the solvent is water, ethanol, propylene glycol, triacetin and mixtures thereof.

9. Method according to one of Claims 5 to 8, **characterized in that** the amount of solvent used, expressed by weight relative to the weight of dry matter (vanillin + ethylvanillin), is from 5 to 60%.

10. Method according to Claim 4, **characterized in that** the mixture of vanillin and ethylvanillin used in a molar ratio of 2 is melted, then the molten mixture is cooled by lowering the temperature to 50°C ± 1, then this temperature is maintained until the mixture has solidified completely.

11. Method according to Claim 10, **characterized in that** the vanillin and ethylvanillin used in a molar ratio of 2 are charged, separately or mixed together, and the mixture is heated to a temperature that is selected in the range from 60°C to 90°C and is preferably between 70°C and 80°C.

12. Method according to Claim 11, **characterized in that** said molten mixture is prepared under an atmosphere of inert gas, which is preferably nitrogen.

13. Method according to one of Claims 10 to 12, **characterized in that** the molten mixture is cooled without stirring.

14. Method according to one of Claims 4 to 13, **characterized in that** the compound obtained is formed by a grinding technique.

15. Method according to one of Claims 10 to 12, **characterized in that** the molten mixture is formed by a flake-forming technique.

16. Compound of vanillin and ethylvanillin, **characterized in that** it is obtained by melting a mixture of vanillin and ethylvanillin used in a molar ratio of 2 at a temperature selected in the range from 60°C to 90°C and preferably between 70°C and 80°C followed by cooling of the molten mixture by lowering the temperature to 50°C ± 1, then maintaining this temperature until the mixture has solidified completely.

17. Composition comprising at least one compound of vanillin and ethylvanillin described in one of Claims 1 to 3 and 16 and at least one excipient selected from fats;,fatty alcohols; sugars; polysaccharides; silica; vanillin and ethylvanillin.

18. Composition according to Claim 17, **characterized in that** the excipient is selected from:
- sugars, preferably glucose, sucrose, fructose, galactose, ribose, maltose, sorbitol, mannitol, xylitol, lactitol, maltitol; invert sugars: glucose syrups as well as sucroglycerides derived from fatty oils, preferably copra oil, palm oil, hydrogenated palm oil and hydrogenated soya oil; sucroesters of fatty acids preferably sucrose monopalmitate, sucrose monodistearate and sucrose distearate,
- starches derived notably from wheat, maize, barley, rice, manioc or potato, native, pregelatinized or modified and more particularly amylose-rich native maize starches, pregelatinized maize starches, modified maize starches, modified waxy maize starches, pregelatinized waxy maize starches, modified waxy maize starches in particular OSSA/sodium octenylsuccinate starch,
- starch hydrolyzates,
- dextrins and maltodextrins resulting from the hydrolysis of a starch (wheat, maize) or a potato flour as well as β-cyclodextrins, preferably maltodextrins having a DE below 20, preferably between 5 and 19 and more preferably between 6 and 15,
- cellulose, its ethers, notably methylcellulose, ethylcellulose, methylethylcellulose, hydroxypropylcellulose; or its esters, notably carboxymethylcellulose or carboxyethylcellulose optionally in the sodium-containing form,
- gums such as gum of kappa carrageenan or iota carrageenan, pectin, guar gum, carob gum, and xanthan gum, alginates, gum arabic, acacia gum, agar-agar,
- flours, preferably wheat flour (native or pregel); starches, preferably potato starch,
- gelatin,
- silica,
- an antioxidant preferably vitamin E,
- an emulsifier preferably lecithin,
- vanillin or ethylvanillin.

19. Composition according to either of Claims 17 and 18, **characterized in that** it comprises from 0.1 to 90 wt.% of excipient(s), preferably from 20 to 60 wt.% of excipient(s).

20. Application of the compound described in one of Claims 1 to 3 and 16 or of the composition described in one of Claims 17 to 19 as a flavoring agent in the field of human and animal nutrition, pharmacy, and as perfume, in the industry of cosmetics, perfumes and detergents.

21. Application according to Claim 20, **characterized in that** the compound or the composition of the invention is used, during manufacture of a dough, preferably in a fat, in the area of dry biscuit-making and industrial cake-making; in the field of chocolate manufacture, notably for preparation of bars of chocolate, couverture chocolates or chocolate fillings; in the manufacture of candies of all kinds: sugared almonds, caramels, nougats, hard candy, fondant candies and others; in the dairy products industry and more particularly in flavored and gelified milks, entremets, yoghurts, ices and ice creams; in the production of vanillin sugar, by impregnation of sugar with vanillin; in the preparation of various drinks, preferably grenadine and chocolate drinks; in the preparation of instant drinks such as flavored drinks in powder form, chocolate in powder form or else in instant preparations in the form of powder intended for the manufacture of all kinds of desserts; for denaturing butter.

22. Application according to Claim 20, **characterized in that** the compound or the composition of the invention is used in animal feed, notably for the preparation of meal.

23. Application according to Claim 20, **characterized in that** the compound or the composition of the invention is used as an odor masking agent, notably in the pharmaceutical industry; in the field of cosmetics for the preparation of creams, milks and make-up and other products, as a perfuming base in perfumery and in the field of detergents, notably in soap-making.
